# EUROPEAN PATENT SPECIFICATION

(11) **EP 4 164 555 B1**
(45) Date of publication and mention of the grant of the patent: **06.05.2026**
(21) Application number: 20941411.9
(22) Date of filing: 16.06.2020
(51) Int. Cl.: A61F 2/91, A61F 2/915

(54) **INTRAVASCULAR STENT**
INTRAVASKULÄRER STENT
STENT INTRAVASCULAIRE

(43) Date of publication of application: 19.04.2023
(73) Proprietor: Suzhou Venmed Technology Co., Ltd., Suzhou, Jiangsu 215163 (CN)
(72) Inventor: WANG, Yonggang, Suzhou, Jiangsu 215163 (CN)
(74) Representative: Fidal Innovation
(86) International application number: PCT/CN2020/096311
(87) International publication number: WO 2021/253216

(56) References cited:
- WO-A1-2007/035619
- WO-A1-2019/236900
- CN-A- 105 250 059
- CN-A- 105 726 178
- CN-A- 106 176 003
- CN-U- 203 408 130
- CN-Y- 201 085 705
- US-A1- 2018 168 833
- US-A1- 2019 247 209
- US-B1- 7 060 088

## Description

### Technical Field of the Invention

The present disclosure belongs to the field of medical apparatus and instruments, specifically to an intravascular stent.

### Background of the Invention

Venous disease is most common in vascular surgical diseases, especially lower extremity deep vein thrombosis and lower extremity great saphenous vein varicosity are even more common. And a big reason for the cause of the formation of these two diseases is that it is caused by iliac vein compression. Iliac vein compression syndrome (IVCS) refers to that iliac vein is compressed by iliac artery, leading to intracavity adhesion, stenosis or occlusion, thereby resulting in a series of clinical symptoms of the syndrome, also known as May-Thurner, Cockett syndrome. The incidence of iliac vein compression syndrome is in 20% to 34%.

Venous compression has always been solved by operation in the treatment, which has been difficult to be accepted by patients due to the need for abdominal surgery and large trauma, so the number of cases reported in China is few. It results in that lower extremity swelling of many patients cannot be resolved, and some patients suffer venous thrombosis due to blood stasis.

At present, many Chinese and foreign research reports show that there is few stent designed for the iliac vein compression syndrome. A stent for the treatment of iliac vein stenosis (utility model: 201320053442.3) is woven by nickel-titanium wires into a latticed reticulated tube, the orifice of the front end of the reticulated tube is a bevel, an angle between the bevel and the cross section of the reticulated tube is 40 degrees, the reticulated tube has an oval mark ring thereon, to realize the accurate positioning. However, none of the iliac venous stents is applicable to the iliac veins due to their support strength and effect on blood flow.

US2019/247209A1 discloses a tapering stent. The stent includes a high radial force segment and a highly flexible segment, where the diameters of the high radial force segment and the highly flexible segment are different. For example, the stent may be formed from a tube having varying diameters as it extends distally combined with increased strut density to achieve increased flexibility distally while reducing loss of radial stiffness. The stent may further be placed with an additional stent segment, where the additional stent segment has a radial force similar to the radial force of the highly flexible force segment. The document is not describing a structure formed by two adjacent second repeating elements of a first supporting part wherein closed-loops and open-loops in the axial direction are alternately arranged.

### Summary of the Invention

The technical problem to be solved by the present disclosure is to overcome the disadvantage of the prior art, and to provide intravascular stents of different structures.

To solve the above technical problems, the present disclosure employs the following technical schemes:
An intravascular stent, capable of changing to an expanded state with an expanded second cross-sectional diameter from a compressed state with a first cross-sectional diameter, wherein,
the intravascular stent comprises a positioning segment and a supporting segment connected in the axial direction of the intravascular stent,
the positioning segment comprises a plurality of first repeating elements distributed in the circumferential direction along the intravascular stent,
the supporting segment comprises a first supporting part, a second supporting part and a third supporting part connected successively in the axial direction of the intravascular stent, and the positioning segment is connected with the first supporting part,
   the supporting segment comprises at least five supporting units and at least four connecting units connected in the axial direction of the intravascular stent, the at least five supporting units each comprising a plurality of second repeating elements distributed in the circumferential direction along the intravascular stent, and the at least four connecting units each comprise a plurality of connectors distributed in the circumferential direction along the intravascular stent,
   the structure of the first repeating elements and the second repeating elements are of any shape capable of forming holes, for example V shape, grid shape, rhombus and the like,
   whereby closed loops are formed when the number of the connectors in a row is equal to the number of the second repeating elements of the supporting units in adjacent rows and each second repeating element is connected to a connector and whereby open loops are formed when the number of connectors in a row is equal to or smaller than half of the number of second repeating elements, and one out of two second repeating elements, or less, is connected to a connector,
the structure formed by two adjacent second repeating elements of the first supporting part is alternately arranged by closed-loops and open-loops in the axial direction,
the structure formed by two adjacent second repeating elements of the second supporting part is open-loops,
the structure formed by two adjacent second repeating elements of the third supporting part is closed-loops.

According to an implementation of the present disclosure, the length of the first supporting part accounts for 0.4-0.6 of the total length of the supporting segment.

According to an implementation of the present disclosure, the length of the third supporting part accounts for 0.05-0.15 of the total length of the supporting segment.

Further, the number of closed-loops in each closed-loop unit is 1, and the number of open-loops in each open-loop unit is 1 in the axial direction, so that the structure formed by two adjacent second repeating elements of each supporting unit of the first supporting part is alternately arranged by closed-loop and open-loop in the axial direction.

According to an implementation of the present disclosure, the second repeating elements of the supporting unit located at front ends of the supporting segments are one-to-one correspondingly connected with the second repeating elements of another adjacent supporting unit to form close-loop structures.

According to an implementation of the present disclosure, a front end of the supporting segment is connected with a plurality of first developing units.

Further, the number of the first developing units being 3 to 10.

According to a preferred aspect of the present disclosure, the front end of the second repeating element of the supporting segment is connected with the first developing unit.

In the present disclosure, the first repeating element may be connected with the second repeating element, or may be connected with the connecting unit.

According to an implementation of the present disclosure, a rear end of the supporting segment is connected with a plurality of second developing units.

Further, the number of the second developing units being 3 to 10.

According to an implementation of the present disclosure, a front end of the positioning segment is connected with a plurality of third developing units.

Further, the number of the third developing units being 3 to 10.

According to an implementation of the present disclosure, holes formed by the first repeating elements are larger than holes formed by the second repeating elements in size so as to avoid the formation of thrombosis caused by a small hole of the intravascular stent or effect on the blood flow of the opposite iliac vein caused by the positioning segment.

According to an implementation of the present disclosure, the number of the first repeating elements is less than the number of the second repeating elements of each of the at least two supporting units.

In the present disclosure, the first repeating element may be connected with the second repeating element, or may be connected with the connecting unit.

In the present disclosure, the supporting units are alternate with the connecting units, that is, two ends of the connecting unit are connected with the supporting unit, respectively.

According to an implementation of the present disclosure, an angle between the plane formed by the front ends of the plurality of first repeating elements and the axis of the intravascular stent is 45° ~ 90°.

According to an implementation of the present disclosure, the material of the intravascular stent is one or more selected from stainless steel, memory alloy, nickel-titanium alloy, titanium alloy, tantalum alloy, cobalt-chromium alloy, biodegradable metal, biodegradable polymer, magnesium alloy, pure iron.

According to an implementation of the present disclosure, an angle between an extending direction of the positioning segment and the axis of the intravascular stent is 0° ~ 60°.

According to a preferred aspect of the present disclosure, the length of the first repeating element is 5 - 15 mm, preferably 10 - 15 mm, the length of the supporting segment is 40 - 140 mm, preferably 60 - 120 mm, and the thickness of the intravascular stent is 0.1 - 0.3 mm.

According to an implementation of the present disclosure, the length of the second repeating element is 1 - 7 mm, preferably 2 - 5 mm.

According to an implementation of the present disclosure, the intravascular stent is configured to be used for an iliac vein stent.

In the present disclosure, the side where the positioning segment is located is defined as the front of the intravascular stent.

In the present disclosure, the defined angle is an angle less than 90° formed between a straight line and a plane or between two straight lines.

In the present disclosure, the total length of the intravascular stent is the longest vertical distance between the front end of the first repeating element and the second developing unit. The lengths of the first repeating elements, the second repeating elements and the third repeating elements refer to the vertical distances from the front end to the rear end of each element. The length of the supporting segment refers to the vertical distance between the first developing unit and the second developing unit. The length of the central supporting segment refers to the vertical distance between the first developing unit and the front end of the end supporting segment. The length of the end supporting segment refers to the vertical distance between the rear end of the central supporting segment and the second developing unit.

The beneficial effects of the present disclosure are:

The intravascular stent of the present disclosure is particularly suitable for iliac vein, with good supporting effect for iliac vein and small damage to venous wall, and can effectively avoid forming in-stent secondary thrombosis after intravascular stent implantation. Moreover, the intravascular stent of the present disclosure can be well positioned in the iliac vein to improve the accuracy of the release, and it is simple for operation. The vascular stent of the present disclosure has the advantages of simple structure, convenient production and low cost, and thereby has important practical significance and good prospect in clinical application.

### Brief Description of Drawings

Fig. 1 is a schematic structure diagram of the intravascular stent of Embodiment being unfolded to a plane;
Fig. 2 is an enlarged diagram of the connector in Embodiment;
Fig. 3 is an enlarged diagram of the first repeating element in Embodiment.

### Detailed Description of Embodiment

In the following, the present disclosure is further described in detail combining with specific embodiments, but is not limit to the following embodiments.

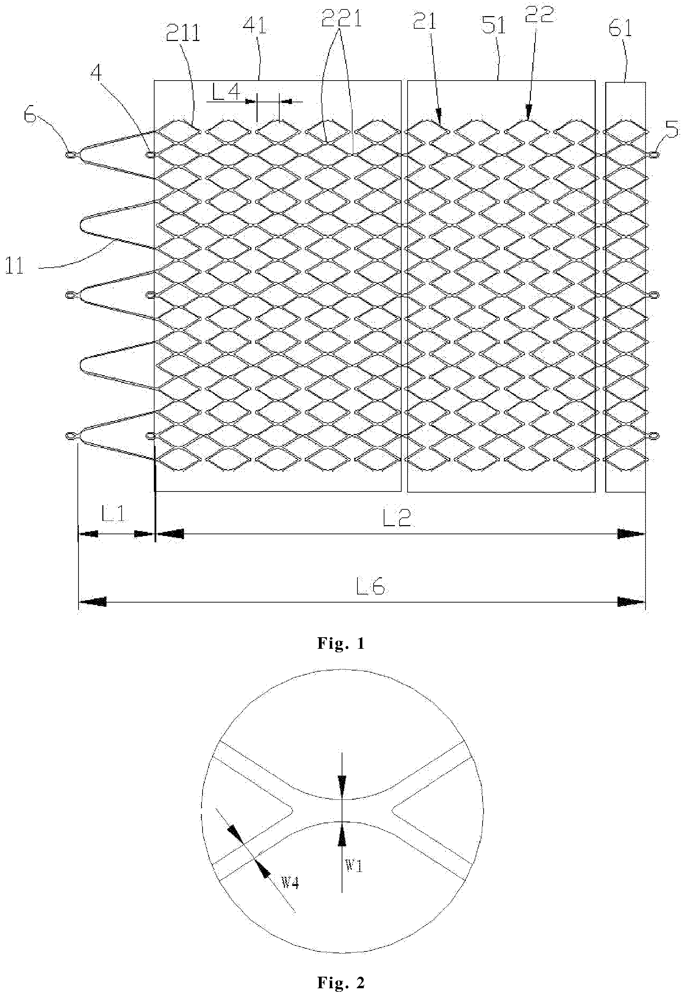

The intravascular stent of the present invention is mainly suitable for bifurcation vessel, in particular for iliac vein (as shown in Figs. 1-3). During implanting the intravascular stent in iliac vein, the intravascular stent is mounted in a conveyor of a stent delivery system, and at present, the intravascular stent is in a compressed state, when the conveyor delivers the intravascular stent to an appropriate position and releases the intravascular stent, the intravascular stent expands outwards to an expanded state and thereby supports iliac vein, the intravascular stent has a first cross section in the compressed state, and a second cross section in the expanded state, and the diameter of the first cross section is less than the diameter of the second cross section. In the present invention, the first cross section and the second cross section are the cross sections of the supporting segment, and the cross section is vertical to the axis of the intravascular stent. In the present invention, the external diameter of the second cross section is 10 - 20 mm, preferably 12 - 16 mm.

The material of the intravascular stent is one or more selected from stainless steel, memory alloy, nickel-titanium alloy, titanium alloy, tantalum alloy, cobalt-chromium alloy, biodegradable metal, biodegradable polymer, magnesium alloy, pure iron. The stainless steel employs SUS-316L stainless steel, or the like. The memory alloy may employ Ni-Ti alloy, Cu-Al-Mn alloy, or the like. The cobalt-chromium alloy may employ CoCr-L605 cobalt-chromium alloy. The biodegradable metal is a metal that would be decomposed in a human body, for example, pure magnesium, magnesium alloy, pure iron, ferroalloy and the like. The biodegradable polymer may be a polymer that would be biodegraded, such as polylactic acid, polyglycolic acid, poly (lactate-ε-caprolactone), poly (glycolic acid-ε-caprolactone) and the like. Moreover, the biodegradable metal may be coated with a biodegradable polymer to serve as intravascular stent material.

The intravascular stent of the present disclosure may be one-step formed via laser engraving. The preparation process of laser engraving is: first, establishing a cutting path by utilizing CAM based on supports design drawing; next, laser cutting the metal or high molecular material; finally, pickling and electrochemical treating to improve the degree of finish of the surface and round the edge shape.

As shown in Figs 1, the intravascular stent comprises a positioning segment and a supporting segment connected from the front to the end in the axial direction of the intravascular stent.

The positioning segment comprises a plurality of first repeating elements 11 distributed in the circumferential direction along the intravascular stent.

The supporting segment comprises at least two supporting units 21 and at least one connecting unit 22 connected in the axial direction of the intravascular stent, the supporting unit 21 comprises a plurality of second repeating elements 211 distributed in the circumferential direction along the intravascular stent, and the connecting unit 22 comprises a plurality of connectors 221 distributed in the circumferential direction along the intravascular stent. The number of the connectors 221 is not more than the number of the second repeating elements 211.

The structures of the first repeating elements 11 and the second repeating elements 211 of the present invention may be any shape capable of forming holes, for example, V shape, grid shape, rhombus and the like. The distribution of the connectors 221 in the whole intravascular stent may be arbitrary, such as linear shaped, V shaped, W shaped, S shaped, N shaped and the like.

In the present invention, a front end of the supporting segment is connected with a first developing unit 4, and in the present embodiment, a front end of the second repeating element 211 of the supporting segment is connected with the first developing unit 4, and the arrangement of the first developing unit 4 causes good positioning of the intravascular stent to facilitate the doctor to find the release point.

In the present invention, the number of the first repeating elements 11 is 4 to 10, and the number of the first developing units 4 and the number of the first repeating elements 11 are equal or not equal.

In the present invention, a rear end of the supporting segment is connected with a plurality of second developing units 5, the number of the second developing units 5 is 3 to 10, and the number of the second developing units 5 and the number of the first developing units 4 are equal. In the present embodiment, the second developing unit 5 may be connected with the supporting unit 21, or with the connecting unit 22, and the second developing units 5 are uniformly distributed in the circumferential direction along the intravascular stent.

In the present invention, the number of the first repeating elements 11 differs from the number of the second repeating elements 211. Preferably, the number of the first repeating elements 11 is less than the number of the second repeating elements 211, and in the present embodiment, the number of the second repeating elements 211 is 2 - 4 times larger than the number of the first repeating elements 11, so as to ensure large hole formed by the first repeating elements 11, and after implanting the intravascular stent in iliac vein, the formation of thrombosis caused by a small hole of the intravascular stent or effect on the blood flow of the opposite iliac vein caused by the positioning segment can be avoid when the positioning segment extends to inferior vena cava.

In the present embodiment, an angle between the extending direction of the positioning segment and the axis of the intravascular stent is 0° ~ 60°. The extending direction of the positioning segment is accordance with the axis of the intravascular stent, that is, the angle is 0°, and in this condition, the intravascular stent in the expanded state is substantially cylinder-shaped. There is an angle between the extending direction of the positioning segment and the axis of the intravascular stent, and the positioning segment extends gradually facing away from the axial direction of the intravascular stent towards the front end from the end connected with the supporting segment, that is, the positioning segment is hydraucone-shaped expanded gradually from rear to front.

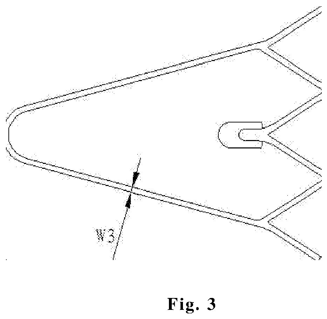

In the present embodiment, the width W1 of the connector 221 (as shown Fig. 2) is 0.1 - 0.3 mm. The width W1 of the connector 221 and the width W3 of the first repeating element 11 (as shown Fig. 3) are equal. The width W4 of the second repeating element 211 (as shown Fig. 2) may employ a conventional width, for example, 0.1 - 0.3 mm. And, the width of the connector 221 is equal to the width of the second repeating element 211.

In the present embodiment, the thickness of the intravascular stent is 0.1 - 0.3 mm (not shown), and the thickness of the intravascular stent refers to the difference between the external diameter and the inner diameter of the cross section of the intravascular stent.

In the present embodiment, the front end of the first repeating element 11 is a rounded corner, and the radius of the inner arc of the rounded corner is larger than 0.1 mm (not shown), so as to reduce the vascular stimulation from the intravascular stent.

In the present embodiment, the length L1 of the first repeating element 11 is 5 - 15 mm, preferably 10 - 15 mm, if the length of the first repeating element 11 is too long, it will cause the front ends of the first repeating elements 11 contact the opposite vessel wall and cause harmful effects such as vascular stimulation; if the length of the first repeating element 11 is too short, it will affect the positioning of the intravascular stent in iliac vein, so as to cause that the intravascular stent cannot be implanted in the desired position stably.

The length L2 of the supporting segment is 40 - 140 mm, preferably 60 - 120 mm, and the length L4 of the second repeating element 211 is 1 - 7 mm, preferably 2 - 5 mm. That is, the supporting segment comprises substantially twenty supporting units 21, of course, the number of the supporting units 21 could be adjusted according to the actual requirement.

The total length L6 of intravascular stent in expanded state of the present invention is 50 - 120 mm, preferably 60 - 100 mm.

The plane formed by the front ends of the plurality of the first repeating elements 11 is vertical to the axis of the intravascular stent(shown in fig.1), or, the plane formed by the front ends of the plurality of the first repeating elements 11 intersects with the axis of the intravascular stent, and the angle between the plane formed by the front ends of the plurality of the first repeating elements 11 and the axis of the intravascular stent is 45° - 89°, wherein, the angle between the plane formed by the front ends of the plurality of the first repeating elements 11 and the axis of the intravascular stent is 70°,and the lengths of the first repeating elements 11 vary gradually, however, the length range thereof should be in the above defined length range of the first repeating elements 11.

A front end of the positioning segment is connected with a plurality of third developing units 6, and the arrangement of the third developing units 6 causes good positioning of the intravascular stent to facilitate the doctor to find the release point. The number of the third developing units 6 is 3 to 10.

The supporting segment comprises a first supporting part 41, a second supporting part 51 and a third supporting part 61 connected successively in the axial direction of the intravascular stent, and the positioning segment is connected with the first supporting part 41, the structure formed by two adjacent second repeating elements 211 of the first supporting part 41 is alternately arranged by closed-loop and open-loop in the axial direction, so that it can well enhance the supporting force of the first supporting part 41 , so as to reduce the incidence of iliac vein stenosis or occlusion.

And the structure formed by two adjacent second repeating elements 211 of the second supporting part 51 is open-loop, so as to reduce the supporting force of the second supporting part 51 and prevent the iliac vein from being pressed by overlarge supporting force.

And the structure formed by two adjacent second repeating elements 211 of the third supporting part 61 is closed-loop. So as to uniformly expand the blood vessel stent; the vascular stent is convenient to install and release, and the stress of the blood vessel is uniform.

In other words, in the first support part 41, when viewed from front to back in the axial direction of the stent, the number of the connectors 221 in an odd-numbered row is equal to the number of the second repeating elements 211 of the supporting units 21 in the adjacent row, and the number of the connectors 221 in an even-numbered row is smaller than the number of the second repeating elements 211 of the supporting units 21 in the adjacent row. For example, the number of the connectors 221 in the first row is equal to the number of the second repeating elements 211 of the supporting units 21 in the adjacent row, and the number of the connectors 221 in the second row is less than the number of the second repeating elements 211 of the supporting units 21 in the adjacent row. So that the structure formed by two adjacent second repeating elements 211 of the first supporting part 41 is alternately arranged by closed-loop and open-loop in the axial direction.

In the second support part 51, the number of the connectors 221 of the connecting unit 22 is less than the number of the second repeating elements 211 of the supporting units 21. So that the structure formed by two adjacent second repeating elements 211 of the second supporting part 51 is open-loop.

In the third support part 61, the number of the connectors 221 in each row is equal to the number of the second repeating elements 211 of the supporting units 21 in the adjacent row. So that the structure formed by two adjacent second repeating elements 211 of the third supporting part 61 is closed-loop.

The length of the first supporting part 41 accounts for 0.4-0.6 of the total length of the supporting segment, the length of the third supporting part 61 accounts for 0.05-0.15 of the total length of the supporting segment.

The above describes the present disclosure in detail, and is intended to make those skilled in the art being able to understand the present disclosure and thereby implement it, and should not be concluded to limit the protective scope as defined by the claims.

## Claims

1. An intravascular stent, capable of changing to an expanded state with an expanded second cross-sectional diameter from a compressed state with a first cross-sectional diameter,
the intravascular stent comprises a positioning segment and a supporting segment connected in the axial direction of the intravascular stent,
the positioning segment comprises a plurality of first repeating elements (11) distributed in the circumferential direction along the intravascular stent,
the supporting segment comprises a first supporting part (41), a second supporting part (51) and a third supporting part (61) connected successively in the axial direction of the intravascular stent, and the positioning segment is connected with the first supporting part (41),
wherein
the supporting segment comprises at least five supporting units (21) and at least four connecting units (22) connected in the axial direction of the intravascular stent, the at least five supporting units (21) each comprising a plurality of second repeating elements (211) distributed in the circumferential direction along the intravascular stent, and the at least four connecting units (22) each comprise a plurality of connectors (221) distributed in the circumferential direction along the intravascular stent,
the structure of the first repeating elements (11) and the second repeating elements (211) are of any shape capable of forming holes, for example V shape, grid shape, rhombus and the like,
whereby closed loops are formed when the number of the connectors (221) in a row is equal to the number of the second repeating elements (211) of the supporting units in adjacent rows and each second repeating element (211) is connected to a connector (221) and whereby open loops are formed when the number of connectors (221) in a row is equal to or smaller than half of the number of second repeating elements (211), and one out of two second repeating elements (211), or less, is connected to a connector (221),
the structure formed by two adjacent second repeating elements (211) of the first supporting part (41) is alternately arranged by closed-loops and open-loops in the axial direction,
the structure formed by two adjacent second repeating elements (211) of the second supporting part (51) is open-loops,
the structure formed by two adjacent second repeating elements (211) of the third supporting part (61) is closed-loops.

2. The intravascular stent according to claim 1, is **characterized in that**, the length of the first supporting part (41) accounts for 0.4-0.6 of the total length of the supporting segment.

3. The intravascular stent according to any one of the preceding claims, is **characterized in that**, the length of the third supporting part (61) accounts for 0.05-0.15 of the total length of the supporting segment.

4. The intravascular stent according to any one of the preceding claims, is **characterized in that**, the second repeating elements (211) of the supporting unit (21) located at front ends of the supporting segments are one-to-one correspondingly connected with the second repeating elements (211) of another adjacent supporting unit (21) to form close-loop structures.

5. The intravascular stent according to any one of the preceding claims, is **characterized in that**, a front end of the supporting segment is connected with a plurality of first developing units (4), the number of the first developing units (4) being 3 to 10.

6. The intravascular stent according to any one of the preceding claims, wherein, a rear end of the supporting segment is connected with a plurality of second developing units (5) , the number of the second developing units (5) being 3 to 10.

7. The intravascular stent according to any one of the preceding claims, is **characterized in that**, a front end of the positioning segment is connected with a plurality of third developing units (6) , the number of the third developing units (6) being 3 to 10.

8. The intravascular stent according to any one of the preceding claims, is **characterized in that**, holes formed by the first repeating elements (11) are larger than holes formed by the second repeating elements (211) in size so as to avoid the formation of thrombosis caused by a small hole of the intravascular stent or effect on the blood flow of the opposite iliac vein caused by the positioning segment.

9. The intravascular stent according to any one of the preceding claims, is **characterized in that**, the number of the first repeating elements (11) is less than the number of the second repeating elements (211) of each of the at least two supporting units (21).

10. The intravascular stent according to any one of the preceding claims, is **characterized in that**, an angle between the plane formed by the front ends of the plurality of first repeating elements (11) and the axis of the intravascular stent is 45° ~ 90°.

11. The intravascular stent according to any one of the preceding claims, is **characterized in that**, the material of the intravascular stent is one or more selected from stainless steel, memory alloy, nickel-titanium alloy, titanium alloy, tantalum alloy, cobalt-chromium alloy, biodegradable metal, biodegradable polymer, magnesium alloy, pure iron.

12. The intravascular stent according to any one of the preceding claims, is **characterized in that**, an angle between an extending direction of the positioning segment and the axis of the intravascular stent is 0° ~ 60°.

13. The intravascular stent according to any one of the preceding claims, is **characterized in that**, the intravascular stent is configured to be used for an iliac vein stent.

## Patentansprüche

1. Intravaskulärer Stent, der in der Lage ist, sich von einem komprimierten Zustand mit einem ersten Querschnittsdurchmesser in einen expandierten Zustand mit einem expandierten zweiten Querschnittsdurchmesser zu verändern,
wobei der intravaskuläre Stent ein Positionierungssegment und ein Stützsegment umfasst, die in der axialen Richtung des intravaskulären Stents miteinander verbunden sind,
wobei das Positionierungssegment eine Vielzahl von ersten Wiederholungselementen (11) umfasst, die in der Umfangsrichtung entlang des intravaskulären Stents verteilt sind,
wobei das Stützsegment einen ersten Stützabschnitt (41), einen zweiten Stützabschnitt (51) und einen dritten Stützabschnitt (61) umfasst, die aufeinanderfolgend in der axialen Richtung des intravaskulären Stents verbunden sind, und wobei das Positionierungssegment mit dem ersten Stützabschnitt (41) verbunden ist,
wobei
das Stützsegment mindestens fünf Stützeinheiten (21) und mindestens vier Verbindungseinheiten (22) umfasst, die in der axialen Richtung des intravaskulären Stents verbunden sind, wobei die mindestens fünf Stützeinheiten (21) jeweils eine Vielzahl von zweiten Wiederholungselementen (211) umfassen, die in der Umfangsrichtung entlang des intravaskulären Stents verteilt sind, und wobei die mindestens vier Verbindungseinheiten (22) jeweils eine Vielzahl von Verbindern (221) umfassen, die in der Umfangsrichtung entlang des intravaskulären Stents verteilt sind,
wobei die Struktur der ersten Wiederholungselemente (11) und der zweiten Wiederholungselemente (211) eine beliebige Form aufweist, die in der Lage ist, Löcher zu bilden, beispielsweise V-Form, Gitterform, Rautenform und dergleichen,
wobei geschlossene Schleifen gebildet werden, wenn die Anzahl der Verbinder (221) in einer Reihe gleich der Anzahl der zweiten Wiederholungselemente (211) der Stützeinheiten in benachbarten Reihen ist und jedes zweite Wiederholungselement (211) mit einem Verbinder (221) verbunden ist, und wobei offene Schleifen gebildet werden, wenn die Anzahl der Verbinder (221) in einer Reihe gleich oder kleiner als die Hälfte der Anzahl der zweiten Wiederholungselemente (211) ist und jedes zweite oder weniger der zweiten Wiederholungselemente (211) mit einem Verbinder (221) verbunden ist,
wobei die durch zwei benachbarte zweite Wiederholungselemente (211) des ersten Stützabschnitts (41) gebildete Struktur abwechselnd durch geschlossene Schleifen und offene Schleifen in der axialen Richtung angeordnet ist,
wobei die durch zwei benachbarte zweite Wiederholungselemente (211) des zweiten Stützabschnitts (51) gebildete Struktur offene Schleifen sind,
wobei die durch zwei benachbarte zweite Wiederholungselemente (211) des dritten Stützabschnitts (61) gebildete Struktur geschlossene Schleifen sind.

2. Intravaskulärer Stent nach Anspruch 1, **dadurch gekennzeichnet, dass** die Länge des ersten Stützabschnitts (41) 0,4-0,6 der Gesamtlänge des Stützsegments beträgt.

3. Intravaskulärer Stent nach einem der vorhergehenden Ansprüche, **dadurch gekennzeichnet, dass** die Länge des dritten Stützabschnitts (61) 0,05-0,15 der Gesamtlänge des Stützsegments beträgt.

4. Intravaskulärer Stent nach einem der vorhergehenden Ansprüche, **dadurch gekennzeichnet, dass** die zweiten Wiederholungselemente (211) der Stützeinheit (21), die sich an den vorderen Enden der Stützsegmente befindet, eins-zu-eins entsprechend mit den zweiten Wiederholungselementen (211) einer anderen benachbarten Stützeinheit (21) verbunden sind, um geschlossene Schleifenstrukturen zu bilden.

5. Intravaskulärer Stent nach einem der vorhergehenden Ansprüche, **dadurch gekennzeichnet, dass** ein vorderes Ende des Stützsegments mit einer Vielzahl von ersten Entwicklungseinheiten (4) verbunden ist, wobei die Anzahl der ersten Entwicklungseinheiten (4) 3 bis 10 beträgt.

6. Intravaskulärer Stent nach einem der vorhergehenden Ansprüche, wobei ein hinteres Ende des Stützsegments mit einer Vielzahl von zweiten Entwicklungseinheiten (5) verbunden ist, wobei die Anzahl der zweiten Entwicklungseinheiten (5) 3 bis 10 beträgt.

7. Intravaskulärer Stent nach einem der vorhergehenden Ansprüche, **dadurch gekennzeichnet, dass** ein vorderes Ende des Positionierungssegments mit einer Vielzahl von dritten Entwicklungseinheiten (6) verbunden ist, wobei die Anzahl der dritten Entwicklungseinheiten (6) 3 bis 10 beträgt.

8. Intravaskulärer Stent nach einem der vorhergehenden Ansprüche, **dadurch gekennzeichnet, dass** die durch die ersten Wiederholungselemente (11) gebildeten Löcher größer als die durch die zweiten Wiederholungselemente (211) gebildeten Löcher sind, um die Bildung einer Thrombose, verursacht durch ein kleines Loch des intravaskulären Stents, oder eine Beeinträchtigung des Blutflusses der gegenüberliegenden Beckenvene, verursacht durch das Positionierungssegment, zu vermeiden.

9. Intravaskulärer Stent nach einem der vorhergehenden Ansprüche, **dadurch gekennzeichnet, dass** die Anzahl der ersten Wiederholungselemente (11) geringer ist als die Anzahl der zweiten Wiederholungselemente (211) jeder der mindestens zwei Stützeinheiten (21).

10. Intravaskulärer Stent nach einem der vorhergehenden Ansprüche, **dadurch gekennzeichnet, dass** ein Winkel zwischen der durch die vorderen Enden der Vielzahl von ersten Wiederholungselementen (11) gebildeten Ebene und der Achse des intravaskulären Stents 45° ~ 90° beträgt.

11. Intravaskulärer Stent nach einem der vorhergehenden Ansprüche, **dadurch gekennzeichnet, dass** das Material des intravaskulären Stents eines oder mehrere ist, ausgewählt aus Edelstahl, Formgedächtnislegierung, Nickel-Titan-Legierung, Titanlegierung, Tantallegierung, Kobalt-Chrom-Legierung, biologisch abbaubarem Metall, biologisch abbaubarem Polymer, Magnesiumlegierung, Reineisen.

12. Intravaskulärer Stent nach einem der vorhergehenden Ansprüche, **dadurch gekennzeichnet, dass** ein Winkel zwischen einer Erstreckungsrichtung des Positionierungssegments und der Achse des intravaskulären Stents 0° ~ 60° beträgt.

13. Intravaskulärer Stent nach einem der vorhergehenden Ansprüche, **dadurch gekennzeichnet, dass** der intravaskuläre Stent dazu konfiguriert ist, als Beckenvenenstent verwendet zu werden.

## Revendications

1. Endoprothèse intravasculaire, capable de passer d'un état comprimé ayant un premier diamètre de section transversale à un état expansé ayant un second diamètre de section transversale expansé, l'endoprothèse intravasculaire comprend un segment de positionnement et un segment de support reliés dans la direction axiale de l'endoprothèse intravasculaire,
le segment de positionnement comprend une pluralité de premiers éléments répétitifs (11) répartis dans la direction circonférentielle le long de l'endoprothèse intravasculaire,
le segment de support comprend une première partie de support (41), une deuxième partie de support (51) et une troisième partie de support (61) reliées successivement dans la direction axiale de l'endoprothèse intravasculaire, et le segment de positionnement est relié à la première partie de support (41),
dans laquelle,
le segment de support comprend au moins cinq unités de support (21) et au moins quatre unités de liaison (22) reliées dans la direction axiale de l'endoprothèse intravasculaire, les au moins cinq unités de support (21) comprenant chacune une pluralité de seconds éléments répétitifs (211) répartis dans la direction circonférentielle le long de l'endoprothèse intravasculaire, et les au moins quatre unités de liaison (22) comprenant chacune une pluralité de connecteurs (221) répartis dans la direction circonférentielle le long de l'endoprothèse intravasculaire,
la structure des premiers éléments répétitifs (11) et des seconds éléments répétitifs (211) est de toute forme capable de former des orifices, par exemple en forme de V, en forme de grille, en forme de losange et similaires,
des boucles fermées étant formées lorsque le nombre de connecteurs (221) dans une rangée est égal au nombre de seconds éléments répétitifs (211) des unités de support dans les rangées adjacentes et que chaque second élément répétitif (211) est relié à un connecteur (221), et des boucles ouvertes étant formées lorsque le nombre de connecteurs (221) dans une rangée est égal ou inférieur à la moitié du nombre de seconds éléments répétitifs (211), et qu'un second élément répétitif (211) sur deux, ou moins, est relié à un connecteur (221),
la structure formée par deux seconds éléments répétitifs (211) adjacents de la première partie de support (41) est agencée en alternance de boucles fermées et de boucles ouvertes dans la direction axiale,
la structure formée par deux seconds éléments répétitifs (211) adjacents de la deuxième partie de support (51) est en boucles ouvertes,
la structure formée par deux seconds éléments répétitifs (211) adjacents de la troisième partie de support (61) est en boucles fermées.

2. Endoprothèse intravasculaire selon la revendication 1, **caractérisée en ce que** la longueur de la première partie de support (41) représente 0,4 à 0,6 de la longueur totale du segment de support.

3. Endoprothèse intravasculaire selon l'une quelconque des revendications précédentes, **caractérisée en ce que** la longueur de la troisième partie de support (61) représente 0,05 à 0,15 de la longueur totale du segment de support.

4. Endoprothèse intravasculaire selon l'une quelconque des revendications précédentes, **caractérisée en ce que** les seconds éléments répétitifs (211) de l'unité de support (21) située aux extrémités avant des segments de support sont reliés en correspondance biunivoque avec les seconds éléments répétitifs (211) d'une autre unité de support (21) adjacente pour former des structures en boucles fermées.

5. Endoprothèse intravasculaire selon l'une quelconque des revendications précédentes, **caractérisée en ce qu'**une extrémité avant du segment de support est reliée à une pluralité de premières unités de développement (4), le nombre des premières unités de développement (4) étant de 3 à 10.

6. Endoprothèse intravasculaire selon l'une quelconque des revendications précédentes, dans laquelle une extrémité arrière du segment de support est reliée à une pluralité de deuxièmes unités de développement (5), le nombre des deuxièmes unités de développement (5) étant de 3 à 10.

7. Endoprothèse intravasculaire selon l'une quelconque des revendications précédentes, **caractérisée en ce qu'**une extrémité avant du segment de positionnement est reliée à une pluralité de troisièmes unités de développement (6), le nombre des troisièmes unités de développement (6) étant de 3 à 10.

8. Endoprothèse intravasculaire selon l'une quelconque des revendications précédentes, **caractérisée en ce que** les orifices formés par les premiers éléments répétitifs (11) sont plus grands en taille que les orifices formés par les seconds éléments répétitifs (211) afin d'éviter la formation de thrombose causée par un petit orifice de l'endoprothèse intravasculaire ou l'effet sur le flux sanguin de la veine iliaque opposée causé par le segment de positionnement.

9. Endoprothèse intravasculaire selon l'une quelconque des revendications précédentes, **caractérisée en ce que** le nombre des premiers éléments répétitifs (11) est inférieur au nombre des seconds éléments répétitifs (211) de chacune des au moins deux unités de support (21).

10. Endoprothèse intravasculaire selon l'une quelconque des revendications précédentes, **caractérisée en ce qu'**un angle entre le plan formé par les extrémités avant de la pluralité de premiers éléments répétitifs (11) et l'axe de l'endoprothèse intravasculaire est de 45° à 90°.

11. Endoprothèse intravasculaire selon l'une quelconque des revendications précédentes, **caractérisée en ce que** le matériau de l'endoprothèse intravasculaire est un ou plusieurs choisis parmi l'acier inoxydable, l'alliage à mémoire de forme, l'alliage nickel-titane, l'alliage de titane, l'alliage de tantale, l'alliage cobalt-chrome, le métal biodégradable, le polymère biodégradable, l'alliage de magnésium, le fer pur.

12. Endoprothèse intravasculaire selon l'une quelconque des revendications précédentes, **caractérisée en ce qu'**un angle entre une direction d'extension du segment de positionnement et l'axe de l'endoprothèse intravasculaire est de 0° à 60°.

13. Endoprothèse intravasculaire selon l'une quelconque des revendications précédentes, **caractérisée en ce que** l'endoprothèse intravasculaire est configurée pour être utilisée en tant qu'endoprothèse de veine iliaque.
